# EUROPEAN PATENT SPECIFICATION

(11) **EP 0 291 612 B1**
(45) Date of publication and mention of the grant of the patent: **08.03.1995**
(21) Application number: 87401572.0
(22) Date of filing: 06.07.1987
(51) Int. Cl.: A61M 5/14

(54) **Implantable pump.**
Implantierbare Pumpe.
Pompe implantable.

(30) Priority: 20.05.1987 US 52471
(43) Date of publication of application: 23.11.1988
(73) Proprietor: Cannon, Robert Lee, III, F-72240 Conlie (FR)
(72) Inventor: Cannon, Robert Lee, III, F-72240 Conlie (FR)
(74) Representative: David, Daniel

(56) References cited:
- EP-A- 0 075 762
- EP-A- 0 202 696
- WO-A-84/01718
- US-A- 4 193 397
- US-A- 4 217 889

## Description

### Background of the invention

### Field of the Invention

This invention relates generally to that class of inventions which includes patient implantable prosthetic devices which offer therapeutic benefits to such patients. More specifically, this invention relates to an implantable, patient-actuated pump system.

### Description of Prior Art

In the broad field of implantable prosthetic devices, one might encounter a wide spectrum of technology ranging from implantable demand heart pacemakers to implanted artificial hip sockets. However, the prior art for the present invention falls within a narrower category of technology relating to implantable pumps.

Companies are today offering implantable pumps in the market place. One particular company offers an expensive pump system that incorporates complicated mechanisms: a pump motor, microelectronics, lithium batteries, external (hand-held) programming devices, etc. The programming devices incorporate radio-frequency transmission to control the pump. While this pump might be attractive as a research device, the cost and complexity of this pump preclude its general therapeutic use.

Another pump available to the market place today consists of two chambers separated by a flexible metal bellows: one chamber acts as a drug reservoir, while the other is the sealed power supply containing a two-phase charging fluid in equilibrium (see US-A-3 731 681). Working with vapor pressures, condensations to the liquid state, etc., this system eliminated need for batteries. But the high technology required to fabricate it makes it also very expensive, and the fluid is again pumped to certain patient locations without any control by the patient and is inherently unresponsive to changing patient needs.

US-A- 4 193 397 teaches the addition of the bolus controlled by the patient.

A third implantable pump system is known (see e.g. EP-A- 202 696) that includes an injection site, a reservoir, a pumping means, and a delivery catheter. The pumping means has a domed body constructed of a resilient elastomeric material with inlet and outlet valves. By exerting an external pressure on the dome, fluid outlet valve is opened and fluid flows to the delivery catheter. The chamber fills again upon release of the external pressure. This pump system, the first at a moderate price, is only suitable for delivery of a metered dose of fluid in a short period of time, in direct response to the patient actuation. It is not adapted for gradual administration of a drug, but only to bolus administration.

The international patent application WO-A-8 401 718 describes an other implantable pump system. It is a fluid handling system for medication infusion system, including reservoir means for holding fluids to be pumped, pump means operating in a pulsatile mode, a fluid accumulator, fluid resistance means or a flow restrictor, and catheter means. However, this system cannot be used to deliver fluid for long durations such as several hours. Indeed, as soon as the pump means is no more activated, the flow rate rapidly decreases to become null. The patient cannot control the application of the substance as regards the fluid out-flow rate while using this implantable pump system.

For many applications it is necessary to administer the drug slowly. Undesirable side effects tend to be related to the maximum concentration of the drug in the bloodstreams and with bolus administration the ratio of maximum concentration to therapeutic concentration is very unfavorable. In the case of narcotics introduced into the cerebro-spinal fluid to control chronic pain, habituation is related to the maximum concentration and again with bolus administration the maximum concentration/therapeutic concentration ratio is unfavorable.

The kinds of therapeutic applications undertaken by these pumping schemes are many. They include application of nitroglycerine for coronary vascular spasm, application of theophyelline for asthma, application of antineoplastic agents (chemotherapy) for cancer, application of lidocaine for cardiac arythmia, application of antimicrobial and antiviral agents for chronic infection (e.g. osteomyelitis), application of morphine and other opiates, endorphines, substance P for chronic intractable pain, and many other applications of other substances/drugs for other medical maladies. In many, if not all of these instances, an improvement would be achieved if the application were made when the patient sensed the need for the substance, and then controlled the application of the substance.

There is a large market for these pump devices. In the United States alone, with respect to the subject of cancer chemotherapy, there are 850,000 new cases per year ; there are 1.8 million persons suffering chronic or intractable pain ; and 0.5 million new cases per year needing chronic antibiotic therapy. All of these human beings would benefit from drug substances that could be pin-point applied to the area of need, and only when needed.

It is thus understood that there are shortcomings in the prior art: this therapy may be provided at varying rates, ad programmable rates, but never in direct response to patient needs, as sensed by the patient for a long period delivery. Applicant has seized upon a solution to this shortcoming, ad to implantable pump shortcomings associated with complicated technology, and discloses and claims herein a significant advance in the art of implantable pumps.

### Summary of the invention

There is provided a pump system with in particular a pump apparatus capable of being implanted within the body of a patient including reservoir means for holding fluid to be pumped, pump means for drawing said fluid from said reservoir and for supplying said fluid, a fluid accumulator, fluid resistance means and catheter means, characterized in that said pump means comprises a resilient pump chamber responsive to application of manual force by said patient in order to deliver fluid to said accumulator and in that said accumulator comprises a chamber adapted to contain up to some defined maximum volume of fluid and which exerts a pressure on that fluid which is independent of the volume contained therein, so that the fluid out-flow rate is predetermined while supplied to said catheter means through said fluid resistance means

The advantage of the accumulator used therein is to regulate the flow of a fluid supplied by a pump means for longer durations, which is desirable to combat certain medical infirmities. For instance, the accumulator includes balloon means for expanding upon receipt of fluid up to a maximum volume and for contracting upon supplying of said fluid to the catheter means, and fluid resistance means hydraulically connected between the output of the balloon and the catheter, In addition, the patient is protected against a dangerous overdose by the maximum volume limit of the accumulator.

Such a pump system has the advantage of uncomplicated and reliable design in combination with self-actuation of the pump under control of the patient in whom such pump is implanted.

### Brief Description of the Drawings

- Fig.1: is a schematic diagram of a pump system without accumulator;
- Fig.2: is a schematic diagram of a first embodiment of the present invention;
- Fig.3: is a schematic diagram of a second embodiment of the present invention;
- Fig.4: is a schematic diagram of a third embodiment of the present invention;
- Fig.5: is a perspective view of the implantable pump;
- Fig 6A: is a section of an embodiment of an accumulator;
- Fig 6B: is a graph showing pressure in relation to volume for a tubular sleeve of compliant material; and,
- Fig 7: is a cross-section of reservoir sac 106 showing cross-sections of hydrophilic, microporous filters and a cross-section of a collecting tube.

### Description of the Preferred Embodiments

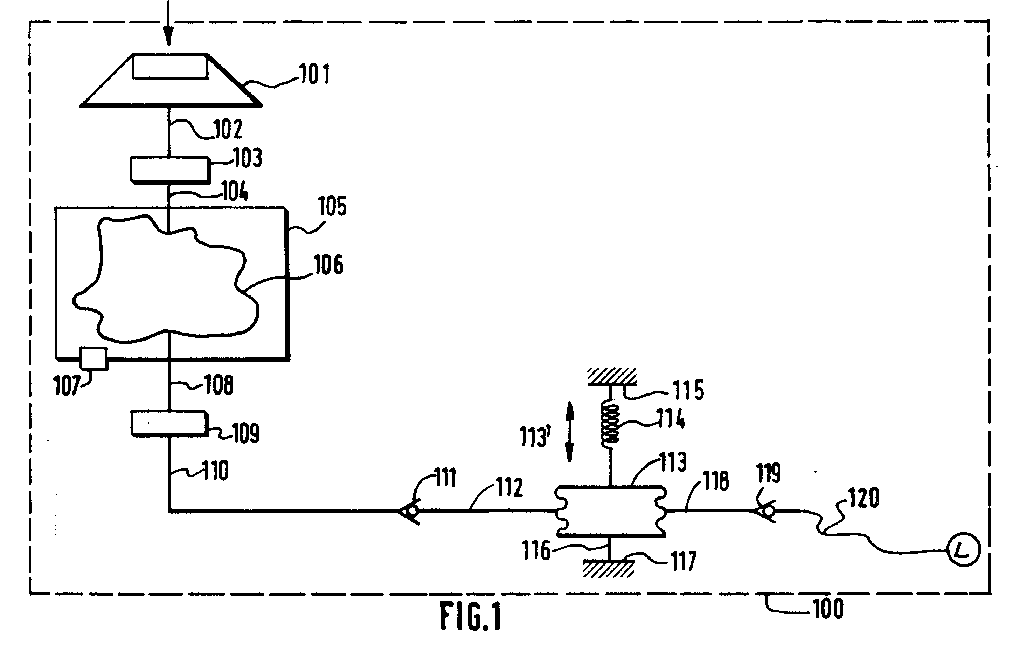

Referring to Fig.1, reference numeral 100 represents the (boundary of the) patient; thus all of the pump system is shown implanted within the patient's body. The pump system is shown in schematic format for ease of explanation of operation.

First, with respect to an interconnection description, septum 101 is connected via fluid conductor 102 through bacterial filter 103 and fluid conductor 104 to collapsible reservoir sac 106 positioned within rigid protective shield/enclosure 105. Bacterial filter 107 permits fluid flow between the patient and the enclosure shield 105 as sac 106 expands/collapses. Fluid from reservoir sac 106 is conducted via fluid conductor 108, bacterial filter 109, fluid conductor 110, one-way valve 111, and fluid conductor 112 to resilient pump chamber 113. Resilient pump chamber 113 is adapted to be compressed in downward direction of arrow 113ʹ, its resilience represented by spring 114 connected between chamber 113 and fixed support 115; fixed support 116/117 is shown connected to the other side of pump chamber 113, the construction of which will be detailed later. The fluid output of pump chamber 113 is provided on fluid conductor 118, through one-way fluid valve 119, to catheter 120, to a particular location "L" within patient 100.

In operation, and continuing to refer to Fig.1, the patient manually depresses resilient chamber 113, thereby forcing fluid therein into fluid conductor 118. Chamber fluid will not return to fluid conductor 112, because of one-way valve 111. This fluid flows through one-way valve 119 and through catheter 120 into the patient. The volume of fluid displaced is no greater than the volume of fluid in the chamber. The pump chamber has a preformed shape and a predetermined maximum chamber volume. The patient may prefer to not depress the chamber completely, and therefore less than the maximum volume of the fluid will be supplied to the patient.

When the patient removes the compressing force on chamber 113, its resilience causes it to return to its preformed shape as it sucks more fluid from reservoir sac 106, (and not from catheter 120 because of the orientation of one-way valve 119).

After pump chamber 113 has been refilled, pump chamber 113 can once again be depressed by the patient, to cause discharge of pump chamber fluid into the patient. This procedure can be repeated as long as fluid from reservoir sac 106 is sufficient to refill pump chamber 113.

If reservoir sac 106 is empty, and if chamber 113 is empty, then the pump system cannot provide additional fluid to the patient unless the reservoir is refilled. Refilling is accomplished by use of a self-sealing septum 101 designed to receive a needle through the skin surface of the patient thus establishing a temporary connection to a syringe. This refilling operation would typically be performed by a physician or other skilled medical practitioner. The fluid from the syringe thus passes through septum 101, fluid conducteur 102, bacterial filter 103, fluid conductor 104, into reservoir sac 106. As sac 106 collapses, bacterial filter 107 passes lymph fluid from the patient into the rigid enclosure 105 to maintain pressure on the fluid in the sac relatively constant; and, as reservoir sac 106 is refilled, the lymph inside of enclosure 105 is forced out through bacterial filter 107 into the patient's body.

Rigid enclosure 105 is desirable to protect and make "puncture-proof" collapsible sac 106; if the needle from the refilling syringe were to puncture sac 106, the fluid leakage therefrom would be uncontrollable and could be harmful to the patient. (Although the pump chamber 113 is resilient, it must likewise be puncture-proof, accomplished by incorporating a titanium or molded-epoxy shield on the outer surface of the chamber). Rigid enclosure 105 also serves as fixed supports 115 and 117. Bacterial filters are required to keep bacterial levels to safe ranges.

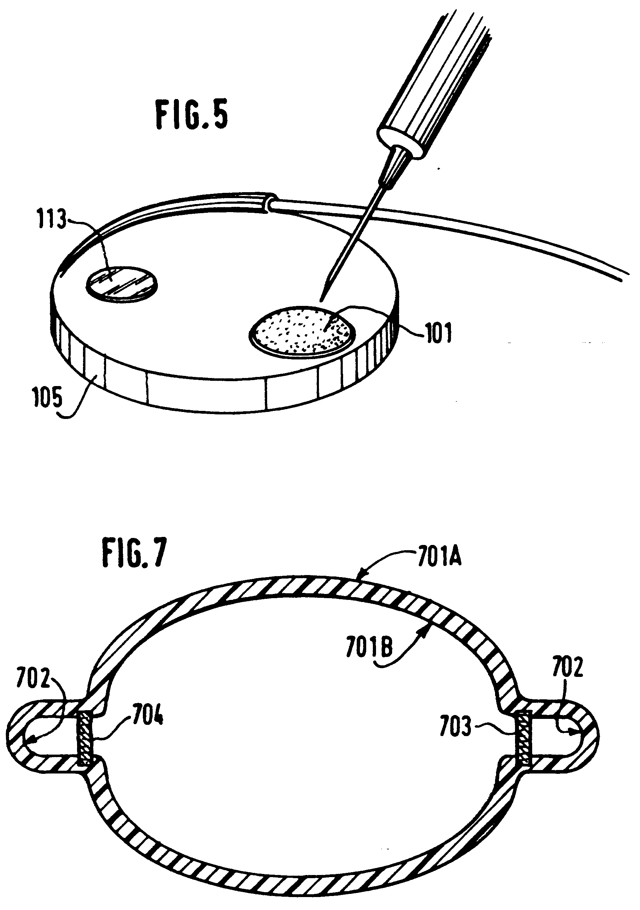

Referring to Fig.5, the shell or enclosure 105 would typically be constructed from titanium or epoxy, and sac 106 (not visible on Fig.5) would be constructed of silicone rubber or polyurethane. The outer dimensions of the shell would be about 50 millimeters diameter, by about 15 millimeters depth. The pump chamber 113 would be protruding above the surface of the enclosure to facilitate for patient actuation; but the reservoir sac would be securely contained internally to the enclosure. Septum 101 is likewise protruding above the surface of container 105, easily accessed by a syringe needle. The maximum volume of typical pump chambers can be designed to range between 50 micro-liters and 250 micro-liters.

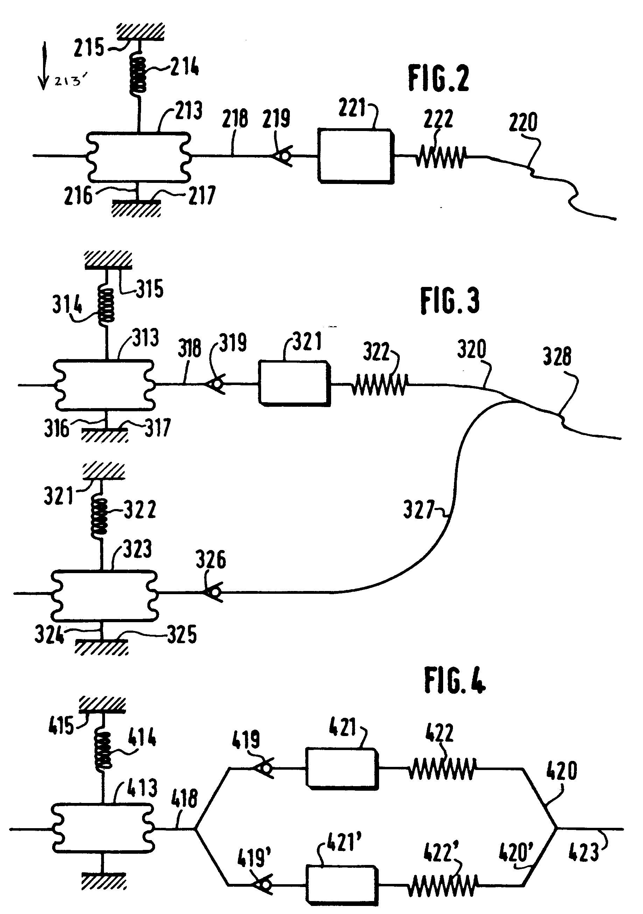

Referring next Fig.2, it is to be understood that the equivalent of the apparatus of Fig 1 connected to the input to pump chamber 113 is assumed to be connected to the input to pump chamber 213 and is not shown for enhancement of clarity of presentation. Spring 214 and fixed supports 215 and 216/217 are equivalent to spring 114 and fixed supports 115 and 116/117 respectively. Pump chamber 213, is resilient as expressed pictorially by spring 214, and moves in downward direction 213ʹ, responsive to manual force exerted by the patient. One way valve 219 permits fluid to flow only from chamber 213 via fluid conductor 218 into accumulator 221, and not in the reverse direction. The fluid output of accumulator 221 is conducted through fluid resistance 222 to catheter 220 and to a specific location within the patient.

According to the invention, accumulator 221 - also named windkessel in view of the function of it - is a chamber adapted to contain up to some defined maximum volume of fluid and which exerts a pressure on that fluid which is an arbitrary, defined function of the volume contained. In the simpliest case, the pressure volume function is such that the pressure is constant, independent of volume. The windkessel fills with in-flow of fluid from pump chamber 213, via valve 219, and empties slowly with out-flow of fluid through resistance 222. The value of resistance 222, and the pressure developed by the windkessel, and other influences, control the out-flow rate. (The flow is to some extent dependent upon pressure in the windkessel, viscosity of the drug, size of the orifice, etc.). The effect of employing windkessel 221 is to provide a dosage of fluid to a specific location within the patient over a prolonged period of time, perhaps on the order of many hours. This long lasting application of fluid may be particularly beneficial in certain chemo-therapy applications for cancer patients, or in other intractable pain situations where the anaesthetic is needed for longer durations.

A windkessel can be constructed in many ways, as:
- a prestretched balloon in a rigid enclosure - to achieve an arbitrary pressure/volume relationship one could vary the thickness, modulus, or manner of prestretching of the balloon;
- a sac compressed by a spring with an arbitrary force/displacement relationship, for instance, constant;
- a cylinder and piston - one would have to use a rolling seal - with a spring with an arbitrary force/displacement relationship, for instance, constant, driving the piston into the cylinder;
- a divided chamber with the divider very compliant and a two-phase (gas - liquid) material on the closed side.

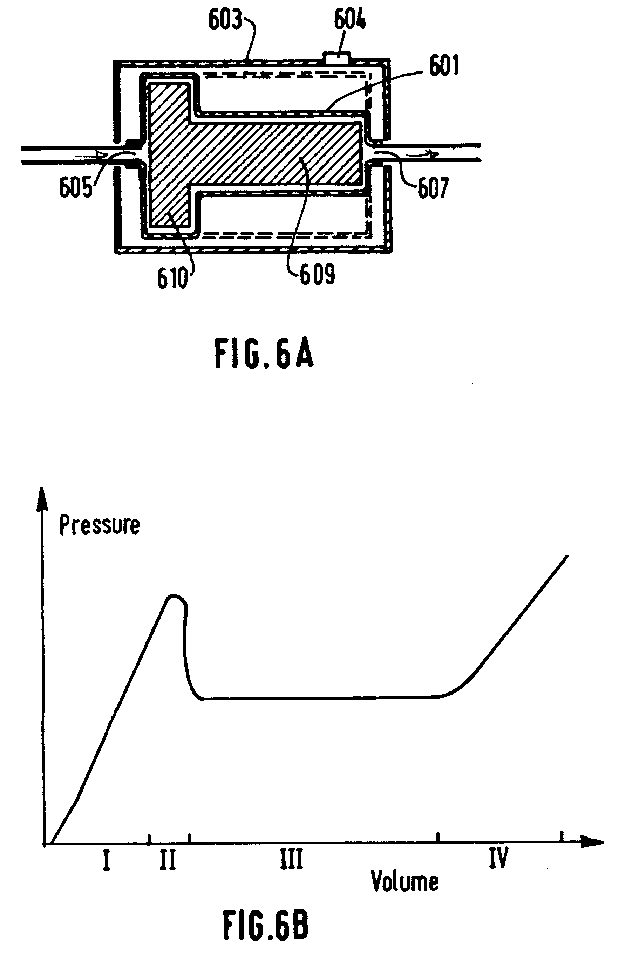

In a particular embodiment, the windkessel is a balloon-like mechanism with resilience, such that the pressure it exerts is essentially independant of the volume it contains. It is constructed (see Fig 6A) as a prestretched sleeve 601 of silicone rubber which is enclosed in a larger rigid tube 603 with a vent 604 to set a maximum limit on the volume of drug in the windkessel. The sleeve 601 is provided with an inlet 605 and an outlet 607. An insert 609 inside the sleeve is so shaped that it prestretches the sleeve 601; In particular at one end 610, the sleeve is at the circumference which it has at the working pressure. When the fluid enters the windkessel, it fills progressively, at a determined pressure, the sleeve that deforms until it takes the shape represented in dotted line in Fig 6A.

The length, diameter, inflated diameter, material of the sleeve are chosen in relation with the desired working values of pressure and volume.

Fig 6B shows a graph giving the relation between pressure and volume inside a tubular sleeve:
- In interval I: the sleeve expands uniformely, the shape remains tubular;
- In interval II: the sleeve expands locally with a sudden rise of diameter at this point. The pressure falls correlatively;
- In interval III: the sleeve expands progressively, while the pressure remains constant ;
- In interval IV: the sleeve is again tubular it expands uniformely until it breaks.

This graph shows that by appropriately prestretching the sleeve it is possible to have the windkessel work in interval III.

Typical values are a windkessel volume of 120 micro-liters and pressure of 100 mm Hg, a 1.40 meter capillary of 80 micro-meters inside diameter, and a drug viscosity of 7 cp. This delivers the 120 micro-liters at a substantially constant rate over an 8 hour period. If the pump-chamber is reactuated prematurely, it simply refills the windkessel.

It is possible to construct the windkessel as either an integral part of the implantable pump or as a component in series with the catheter of an existing implantable pump.

Referring next to Fig 3, a configuration is shown which is particularly suitable for diabetic patients needing insulin. Again, it is to be understood that the equivalent of apparatus of Fig 1 connected to input of pump chamber 113 is assumed to be connected to each input of pump chamber 313 and 323, thus providing proper fluid or insulin supply from the reservoir to both chambers 313 and 323. This input apparatus including the reservoir is not shown, to enhance clarity of presentation.

Springs 314 and 322 and fixed supports 315, 316/317, 321, 324/325 are equivalent to their earlier described counterparts.

Pump chambers 313 and 323 are resilient, and are each operable as earlier described. Fluid output of chamber 313 passes through one way valve 319 to windkessel 321, the output of which is conducted to catheter 328. Fluid output of chamber 323 passes through one-way valve 326 and directly thereafter to catheter 328. For diabetics, where a long steady dosage of insulin (via windkessel 321) is desirable, supplemented by as-needed dosages of insulin (from chamber 323) over and above the steady insulin stream, this design is particularly useful. Choice of resistance value, and chamber sizes can control the ratio of "steady flow" or "background" of insulin to "pulsated" or "supplementary" insulin, as may be desired for any particular patient.

Referring to Fig 4, a dual-rate infusion pump is schematically represented by two windkessels connected from a common chamber, as shown. Again, it is to be understood that the equivalent of apparatus of Fig 1 connected to input of pump chamber 113 is assumed to be connected to the input of pump chamber 413, thus providing proper fluid supply from the reservoir to chamber 413. The input apparatus is again not shown, to enhance clarity of presentation. Spring 414 and fixed supports 415, 416/417 are equivalent to their earlier described counterparts. Pump chamber 413 is resilient and is operable as earlier described. Fluid output of chamber 413 is directed to windkessel 421 through one way valve 419, and to windkessel 421ʹ through one way valve 419ʹ. Output of windkessel 421 is directed through resistance 422 to catheter 428, and output of windkessel 421ʹ is directed through fluid resistance 422ʹ to catheter 428. By judicious selection of resistance values, windkessel volumes and coefficients of elasticity, one can design a system whereby two flow rates into the catheter are achieved. For example, windkessel 421 can provide a 24 hour flow and windkessel 421ʹ can provide a six hour flow on top of the flow from windkessel 421. This dual rate infusion pump has particular application in cancer chemotherapy with anti-neoplastic agents having a significant diurnal variation in activity.

Referring to Fig 7, there is shown a cross-section of the wall of reservoir sac 106 depicted in Fig 1. Outer surface 701A and inner surface 701B of the reservoir sac wall are continuous and form a volume internal to inner surface 701B capable of containing fluid employed in the present invention. Surface 702, although a continuation of inner surface 701B, is also a cross-section of the inner surface of a collection tube. The tube (not shown) runs around the periphery of sac 106. Fluid from inside the sac passes into the collection tube through hydrophilic, microporous filters 703 and 704, (constructed from a particular type of filter paper in a specific embodiment of the invention) which permit fluid but not air to pass. The collection tube has multiple connections to the fluid in sac 106 through multiple filters which can be spaced at regular intervals around the periphery of the sac. The cross-sections of only two such filters are shown herein. The tube is connected through bacterial filter 109 (Fig 1) and resistance 111 (Fig 1) to pump chamber 113 (Fig 1). The reason for the hydrophilic, microporous filters is to prevent air which has entered the reservoir inadvertently from flowing into the pump chamber.

## Claims

1. A pump system with in particular a pump apparatus capable of being implanted within the body of a patient including reservoir means (105, 106) for holding fluid to be pumped, pump means (113, 114, 115, 116, 117) for drawing said fluid from said reservoir (105, 106) and for supplying said fluid, a fluid accumulator, fluid resistance means (222) and catheter means (120), characterized in that said pump means comprises a resilient pump chamber responsive to application of manual force by said patient in order to deliver fluid to said accumulator and in that said accumulator comprises a chamber adapted to contain up to some defined maximum volume of fluid and which exerts a pressure on that fluid which is independent of the volume contained therein, so that the fluid out-flow rate is predetermined while supplied to said catheter means (120) through said fluid resistance means (222).

2. The pump system according to claim 1, characterized in that said chamber is a sac compressed by a spring with a predetermined force.

3. The pump system according to claim 1, characterized in that said chamber includes balloon means comprising prestretched sleeve (601) enclosed in a larger rigid tube (603), the prestretched sleeve (601) being adapted to expand up to a maximum volume of fluid while exerting a predetermined pressure to the fluid contained therein, so that the fluid out-flow rate is predetermined.

4. The pump system according to one of the preceeding claims, characterized in that it comprises pump means (113, 114, 115, 116, 117) for drawing said fluid from said reservoir (105, 106) and for supplying said fluid through catheter means (120) to specific locations within the body of said patient, said pump means (113-117) including a pump chamber (113) having a pre-formed shape and a maximum volume and being hydraulically connected from said reservoir means (105, 106) for receiving up to said maximum volume of fluid from said reservoir means (105, 106).

5. The pump system according to claim 4, characterized in that said pump chamber (113) is constructed from resilient material so that said pump chamber (113) collapses responsive to said application of manual force, and reforms to said pre-formed shape responsive to lack of said application of manual force whereby said pump chamber (113) reacquires said predetermined maximum volume of said fluid from said reservoir means (105, 106) to which said pump chamber (113) is hydraulically connected.

6. The pump system according to claim 4, characterized in that said catheter means (120) includes valve means (119) for controlling flow of said no more than said maximum volume of said fluid to be unidirectional away from said pump chamber (113) and towards said particular location.

7. The pump system according to claim 4 or 5, characterized in that said reservoir means (105, 106) includes a collapsible sac (106) for holding said fluid and for automatically adjusting its volume to be equal to the volume of said fluid.

8. The pump system according to claim 7, characterized in that said reservoir means (105, 106) further includes :
a) protective encapsulation means (105) for containing and protecting said collapsible sac (106), and
b) septum means (101) hydraulically connected to said sac (106), and physically connected to said protective encapsulation means (105), for receiving resupply of said fluid by way of a needle through the skin surface of said patient into said sac (106).

9. The pump system according to claim 8, characterized in that a first bacterial filter (103) is hydraulically located between said septum (101) and input to said sac (106), and wherein a second bacterial filter (109) is hydraulically located between output from said sac (106) and said pump chamber (113).

10. The pump system according to claim 8, characterized in that said reservoir means (105, 106) includes hydrophilic, microporous filter means (107), fluid flowing from said reservoir means (105, 106) passing through said filter means (107).

11. The pump system according to one of the preceeding claims 4 to 10, characterized in that it further comprises a second accumulator including balloon means (421') receiving up to said maximum volume of said fluid from said pump means (413, 414, 415) and fluid resistance means (442') hydraulically connected to the output of said balloon means (421') for supplying said fluid through catheter means (428) at a second predetermined flow rate different from the one provided by the first accumulator (421, 422) alone.

12. The pump system according to one of the claims 1 to 10, characterized in that it further comprises second pump means including a second pump chamber (323) having a second formed shape and a predetermined maximum volume and being hydraulically connected from said reservoir means (105, 106), for receiving up to a second pre-determined maximum volume, and responsive to application of a second manual force by said patient for immediately supplying no more than second maximum volume of said fluid to said catheter (328).

## Patentansprüche

1. Pumpvorrichtung mit im wesentlichen einem in den Körper eines Patienten implantierbaren Pumpapparat, welche Behälter (105, 106) zur Aufnahme der zu pumpenden Flüssigkeit, Pumpeinrichtungen (113, 114, 115, 116, 117) zum Abziehen der Flüssigkeit aus dem Behälter (105, 106) und Weiterleitung derselben, einen Flüssigkeitsakkumulator, Flüssigkeitswiderstandseinrichtungen (222) sowie Kathetereinrichtungen (120) aufweist, dadurch gekennzeichnet, daß die Pumpeinrichtungen eine elastische Pumpe aufweisen, welcheauf manuelle Druckanwendung durch den Patienten reagiert, um dem Akkumulator Flüssigkeit zuzuführen, und daß der Akkumulator eine Kammer aufweist, welche ein bestimmtes maximales Flüssigkeitsvolumen enthalten kann und welch einen Druck auf die Flüssigkeit ausübt, der unabhängig von dem darin enthaltenen Volumen ist, so daß die Geschwindigkeit der ausströmenden Flüssigkeit, während diese den Kathetereinrichtungen (120) über die Flüsigkeitswiderstandseinrichtungen (222) zugeführt wird, vorgegeben ist.

2. Pumpvorrichtung nach Anspruch 1, dadurch gekennzeichnet, daß es sich bei der Kammer um ein, von einer Feder mit einem vorgegebenen Druck zusammengedrücktes, beutelförmiges Behältnis handelt.

3. Pumpvorrichtung nach Anspruch 1, dadurch gekennzeichnet, daß die Kammer Balloneinrichtungen mit einer in einer größeren starren Röhre (603) enthaltenen Hülle (601) aufweist, wobei sich die vorgedehnte Hülle (601) während der Ansübung eines vorgegebenen Druckes auf die darin enthaltene Flüssigkeit bis zu einem maximalen Flüssigkeitsvolumem ausdehnen kann, so daß die Geschwindigkeit der ausströmenden Flüssigkeit vorgegeben ist.

4. Pumpvorrichtung nach einem der vorangegangenen Ansprüche, dadurch gekennzeichnet, daß diese Pumpeinrichtungen (113, 114, 115, 116, 117) zum Abziehen der Flüssigkeit aus dem Behälter (105, 106) und Weiterleitung derselben über Kathetereinrichtungen (120) an bestimmte Stellen im Körper des Patienten aufweist, wobei die Pumpeinrichtungen (113-117) eine Pumpenkammer (113) enthalten, welche eine vorgeformte Form und ein maximales Volumen aufweist und mit dem Behälter (105, 106) hydraulisch verbunden ist, um aus diesem Behälter (105, 106) Flüssigkeit bis zu einem maximalen Volumen aufzunehmen.

5. Pumpvorrichtung nach Anspruch 4, dadurch gekennzeichnet, daß die Pumpenkammer (113) aus einem elastischen Material besteht, so daß sie sich als Reaktion auf manuelle Druckanwendung zusammenzieht und bei unterlassener Druckanwendung wieder ihre vorgeformte Form annimmt, wodurch die Pumpenkammer (113) das vorgegebene maximale Volumen der Flüssigkeit, welche aus dem Behälter (105, 106) stammt, an den die Pumpenkammer hydraulisch angeschlossen ist, wieder erreicht.

6. Pumpvorrichtung nach Anspruch 4, dadurch gekennzeichnet, daß die Kathetereinrichtungen (120) Ventile (119) aufweisen, die den Fluß dahingehend regeln, daß nicht mehr als das maximale Volumen der Flüssigkeit von der Pumpenkammer (113) in lediglich einer Richtung zu der entsprechenden Stelle fließt.

7. Pumpvorrichtung nach Anspruch 4 oder 5, dadurch gekennzeichnet, daß der Behälter (105, 106) ein zusammenziehbares, beutelförmiges Behältnis aufweist, welches die Flüssigkeit aufnimmt und sein Volumen automatisch diesem der Flüssigkeit anpaßt.

8. Pumpvorrichtung nach Anspruch 7, dadurch gekennzeichnet, daß in dem Behälter weiterhin enthalten sind:
a) Schutzeinkapselung (105) zur Aufnahme und zum Schutz des zusammenziehbaren, beutelförmigen Behältnisses (106) und
b) hydraulisch an das beutelförmige Behältnis (106) angeschlossenes und physisch mit der Schutzeinkapselung (105) verbundenes Septum (101) zur Wiederzuführung der Flüssigkeit über eine Nadel durch die Hautoberfläche des Patienten in den beutelförmigen Behälter (106).

9. Pumpvorrichtung nach Anspruch 8, dadurch gekennzeichnet, daß ein erster Bakterienfilter (103) zwischen dem Septum (101) und dem Eingang zu dem beutelförmigen Behältnis und ein zweiter Bakterienfilter (109) zwischen dem Ausgang aus dem beutelförmigen Behältnis (106) und der Pumpenkammer (113) hydraulisch angeordnet ist.

10. Pumpvorrichtung nach Anspruch 8, da durch gekennzeichnet, daß der Behälter (105, 106) einen hydrophilen, mikroporösen Filter (107) aufweist, wo bei die dem Behälter (105, 106) geleitete Flüssigkeit den Filter (107) passiert.

11. Pumpvorrichtung nach einem der vorangegangen Ansprüche 4 bis 10, dadurch gekennzeichnet, daß diese ferner einer zweiten Akkumulator aufweist, welcher einer Balloneinrichtung (421') besitzt, die die ans den Pumpeinrichtungen (413, 414, 415) zugeführte Flüssigkeit bis zu einem maximalen Volumen aufnimmt, sowie eine hydraulisch mit dem Ausgang der Balloneinrichtung (421') verbundene Flüssigkeitswiderstandseinrichtung (442') aufweist, um die Flüssigkeit über die Kathetereinrichtung (428) mit einer zweiten vorgegebenen Fließgeschwindigkeit, welche sich von der, seitens des ersten Akkumulators (421, 422) vorgesehenen Geschwindigkeit unterscheidet, weiterzuleiten.

12. Pumpvorrichtung nach einem der Ansprüche 1 bis 10, dadurch gekennzeichnet, daß diese weiterhin eine zweite, eine zweite Pumpenkammer (323) enthaltende Pumpeinrichtung aufweist, welche eine zweite vorgeformte Form und ein vorgegebenes maximales Volumen besitzt und hydraulisch mit dem Behälter (105, 106) verbunden ist, um Flüssigkeit bis zu einem vorgegebenen zweiten, maximalen Volumen aufzunehmen und, in Reaktion auf eine zweite manuelle Druckanwendung durch den Patienten, dem Katheter (328) nicht mehr Flüssigkeit als durch das zweite maximale Volumen vorgegeben zuzuführen.

## Revendications

1. Un système de pompe avec en particulier un appareil de pompe capable d'être implanté dans le corps du patient incluant un réservoir (105,106) pour contenir un fluide qui sera pompé, une pompe (113,114,115,116,117) pour conduire ledit fluide dudit réservoir (105,106) et pour fournir ledit fluide, un accumulateur de fluide, une résistance (222) et un cathéter (120) caractérisé en ce que ladite pompe comprend une chambre de pompe résistante répondant à l'application d'une force manuelle par ledit patient dans le but délivrer un fluide audit accumulateur et en ce que le dit accumulateur comprend une chambre adaptée pour contenir un volume maximum défini de fluide et qui exerce une pression sur ce fluide indépendante du volume contenu, si bien que le débit sortant est prédéterminé pendant qu'il est fourni au dit cathéter (120) au travers de la résistance (122).

2. Système de pompe selon la revendication 1, caractérisé en ce que ladite chambre est un sac comprimé par un ressort avec une force prédéterminée.

3. Système de pompe selon la revendication 1, caractérisé en ce que ladite chambre inclut un ballon comprenant une gaine (601) prétendue enfermée dans un tube rigide plus large (603), la gaine prétendue (601) étant adaptée à s'expanser au volume maximum de fluide tout en exerçant une pression prédéterminée sur le fluide qu'elle contient, si bien que le débit sortant est prédéterminé.

4. Système de pompe selon l'une des revendications précédentes, caractérisé en ce qu'il comprend une pompe (113,114,115,116,117) pour aspirer ledit fluide dudit réservoir (105,106) et pour fournir ledit fluide au travers du cathéter (120) en un endroit spécifique l'intérieur du corps dudit patient, ladite pompe (113-117) incluant une chambre pompe (113) préformée ayant un volume maximum et étant hydrauliquement connectée au dit réservoir (105,106) pour recevoir ledit volume maximum dudit réservoir (105,106).

5. Système de pompe selon la revendication 4, caractérisé en ce que ladite chambre pompe est fabriquée dans un matériau élastique si bien que ladite pompe (113) s'écrase sous ladite application d'une force manuelle et reprend ladite préforme en réponse au retrait de ladite application de la force manuelle par quoi la chambre pompe (113) réacquiert ledit volume maximum prédéterminé dudit fluide dudit réservoir (105,106) auquel ladite chambre (113) est hydrauliquement connectée.

6. Système de pompe selon la revendication 4, caractérisé en ce que ledit cathéter (120) inclut une valve (119) pour contrôler le flux d'un volume pas plus important que ledit volume maximum dudit fluide pour être unidirectionnellement dirigé de ladite chambre pompe et vers ledit lieu particulier.

7. Système de pompe selon la revendication 4 ou 5, caractérisé en ce que ledit réservoir (105,106) inclut un sac déformable (106) pour contenir ledit fluide et pour ajuster automatiquement son volume pour qu'il soit égal au volume dudit fluide.

8. Système de pompe selon la revendication 7, caractérisé en ce que ledit réservoir (105,106) inclut de plus :
a) Une enceinte protectrice (105) pour contenir et protéger ledit sac déformable (106)
b) Un septum (101) hydrauliquement connecté au dit sac (106) et physiquement connecté à ladite enceinte protectrice (105) pour recevoir le réapprovisionnement dudit fluide au moyen d'une aiguille au travers de la peau dudit patient dans ledit sac (106).

9. Système de pompe selon la revendication 8, caractérisé en ce que le premier filtre bactérien (103) est hydrauliquement localisé entre ledit système de septum (101) et à l'entrée dudit sac (106) et en ce qu'un second filtre bactérien (109) est hydrauliquement localisé entre la sortie dudit sac (106) et ladite chambre (113).

10. Système de pompe selon la revendication 8, caractérisé en ce que ledit réservoir (105,106) inclut un filtre microporeux hydrophile (107), le fluide s'écoulant dudit réservoir (105,106) passant au travers dudit filtre (107)

11. Système de pompe selon l'une des revendications 4 à 10, caractérisé en ce qu'il comprend de plus un second accumulateur incluant un ballon (421) recevant ledit volume maximum dudit fluide de ladite pompe (413,414,415) et une résistance (442) hydrauliquement connectée à la sortie dudit ballon (421) pour fournir ledit fluide au travers du cathéter (428) à un second prédéterminé débit différent de celui fourni par le premier accumulateur (421,422).

12. Système de pompe selon l'une des revendications 1 à 10, caractérisé en ce qu'il comprend de plus une seconde pompe incluant une seconde chambre pompe (323) ayant une seconde forme et un volume maximum prédéterminé et étant hydrauliquement connectée au dit réservoir (105,106) pour recevoir un second volume maximum prédéterminé et répondre à l'application d'une seconde force manuelle dudit patient pour fournir immédiatement pas plus que le second volume maximum dudit fluide au dit cathéter (328).
